**Europäisches Patentamt**

**European Patent Office**

**Office européen des brevets**

(19)

(11) Veröffentlichungsnummer: **0 141 820**
**B1**

(12) **EUROPÄISCHE PATENTSCHRIFT**

(45) Veröffentlichungstag der Patentschrift:
**01.04.87**

(51) Int. Cl.⁴: **A 61 F 2/30**

(21) Anmeldenummer: **84900375.1**

(22) Anmeldetag: **10.01.84**

(86) Internationale Anmeldenummer:
**PCT/CH 84/00003**

(87) Internationale Veröffentlichungsnummer:
**WO 84/03037 (16.08.84** Gazette 84/20)

(54) **GERADER, BLATTARTIGER SCHAFT FÜR EINE GELENKENDOPROTHESE.**

(30) Priorität: **10.02.83 CH 748/83**

(43) Veröffentlichungstag der Anmeldung:
**22.05.85 Patentblatt 85/21**

(45) Bekanntmachung des Hinweises auf die Patenterteilung:
**01.04.87 Patentblatt 87/14**

(84) Benannte Vertragsstaaten:
**AT DE FR GB**

(56) Entgegenhaltungen:
**EP - A - 0 041 591**
**EP - A - 0 058 745**
**EP - A - 0 085 147**
**FR - A - 1 278 359**
**US - E - 28 895**

(73) Patentinhaber: **GEBRÜDER SULZER AKTIENGESELLSCHAFT, Zürcherstrasse 9, CH-8401 Winterthur (CH)**

(72) Erfinder: **GANZ, Reinhold, Walchstrasse 10, CH-3073 Gümlinge (CH)**
Erfinder: **NIEDERER, Peter, Gino, P.O. Box 31178, St. Barbara, CA 93130 (US)**
Erfinder: **FREY, Otto, Wallrütlstrasse 56, CH-8400 Winterthur (CH)**

(74) Vertreter: **Dipl.-Ing. H. Marsch Dipl.-Ing. K. Sparing Dipl.-Phys.Dr. W.H. Röhl Patentanwälte, Rethelstrasse 123, D-4000 Düsseldorf (DE)**

### Beschreibung

Die Erfindung betrifft einen geraden, blattartigen Schaft für eine Gelenkendoprothese, insbesondere eine Hüftgelenkprothese, dessen Blattseiten sich vom distalen freien Ende zunächst symmetrisch zu einer Längsmittelachse konisch erweitern, ehe die laterale Schmalseite auf etwa 2/3 bis 3/4 der Schafthöhe — gemessen entlang der Längsmittelachse vom distalen Ende bis zu einer im wesentlichen horizontalen, zum Prothesenhals führenden Schulter — parallel zur Längsmittelachse oder auf sie zu verläuft, während die mediale Schmalseite in einem Bogen zum Prothesenhals geführt ist.

Prothesenschäfte der vorstehend genannten Art sind bekannt (EP-A-0 058 745). Sie sind vor allem für die Verankerung des Femurteils von Hüftgelenksprothesen bestimmt. Die Wirkung dieser sogenannten Geradschäfte beruht zu einem wesentlichen Teil darauf, dass sie sich in einem auf ihre Dimensionen abgestimmten Hohlraum der Markhöhle verklemmen, wodurch ein sie gegebenenfalls umschliessender Köcher aus Knochzement — bei der zementarmen Verankerung — weitgehend von tragenden Funktionen entlastet wird. Die die Belastungen aufnehmende Abstützung und die Weiterleitung der Kräfte erfolgt dann in erster Linie durch Verklemmen des Schaftblattes medial und lateral an der Kortikalis des Knochens und durch Anpassen des stetig gekrümmten Bogens der medialen Schmalseite an den im Femur medial gelegenen Calcar-Bogen. Sowohl bei zementfreier als auch bei zementarmer Verankerung stehen dabei die mediale und die laterale Schmalseite des Prothesenschaftes in direktem Kontakt mit den Wänden des operativ ausgeräumten Knochens.

Da die ausgeräumte Markhöhle häufig eine unregelmässige Form hat, hat sich in der Praxis eine Anfälligkeit der so verankerten Prothesenschäfte gegen Rotationsbewegungen im Knochen gezeigt, vor allem bei zementfreien Verankerungen in der Anfangsphase nach der Implantation, wenn noch kein neues Knochengewebe an den Schaft angewachsen ist.

Aus der EP-B-0 041 591 ist daher eine Drehsicherung bekannt, bei der trapezförmige Scheibenkörper verschiebbar in Durchbrüchen des Schaftkörpers gelagert sind. Diese Durchbrüche bewirken unter Umständen eine unzulässige Beeinträchtigung der Festigkeit der Schaftkörper; weiterhin sind das Implantieren des Schaftes und das Fixieren der Sicherungselemente erschwert, weil die Scheiben vor dem Einschlagen des Schaftes in den Femur im Schaft «montiert» werden müssen.

Aufgabe der Erfindung ist es, einen Schaft der eingangs genannten Art zu schaffen, der ohne Schwächung seiner Festigkeit eine erhöhte Sicherheit gegen Rotation im Knochen hat, wobei die Möglichkeit vorhanden sein sollte, die Rotationssicherung an unterschiedliche «lichte» Weiten zwischen den kortikalen Wänden des Femurs anzupassen. Diese Aufgabe wird mit der Erfindung dadurch gelöst, dass in den proximalen Bereich des Schaftes lateral des Prothesenhalses in den Blattseiten unter einem Winkel zur Längsmittelachse verlaufende Führungen vorgesehen sind, in die keilförmige Klemmkörper eingeschoben sind, deren Länge mindestens das 1$^1/_2$fache der Länge der Führungen beträgt.

Mit Hilfe der Klemmkörper, die in die Führungen eingetrieben oder eingeschlagen werden, kann der Schaft durch den Operateur in einer vorbestimmten Höhen- und Winkellage gehalten und so verkeilt werden, dass er gegen Rotation im Knochen gesichert ist; die Länge der Klemmkörper beträgt mindestens die 1$^1/_2$fache Länge der Führung, so dass der Klemmkörper jeweils durch Verwendung eines mehr oder weniger tiefen Teiles an die lichte Weite zwischen dem «Boden» der Führung und der gegenüberliegenden Wand der Kortikalis in gewissem Umfang angepasst werden kann. Selbstverständlich ist es möglich, jedem Schaft mehrere Klemmkörper unterschiedlicher Breite beizufügen. Die Klemmkörper können sowohl in spongiöses Gewebe als auch in ein Knochenzementbett eingebracht werden. Die Führungen können dabei als Vertiefungen in die Schaftblätter eingelassen sein; soll jedoch der Schaft in seiner Festigkeit nicht durch Querschnittsverminderungen beeinträchtigt werden, so ist es vorteilhaft, wenn die Führungen als aus der Blattoberfläche herausragende Vorsprünge ausgebildet sind. Dabei können die Vosrprünge, ähnlich parallel zueinander verlaufenden Schienen, durchgehend ausgebildet sein oder auch aus einzelnen links und rechts einer Geraden untereinander fluchtend verteilten Höckern bestehen. Weiterhin können die Führungen bezüglich einer den Blattseiten parallelen Mittel- oder Symmetrieebene des Schaftes einander senkrecht gegenüberliegend oder versetzt zueinander in oder auf den Blattseiten verlaufen.

Während die Schäfte in bewährter Weise aus einem der in der Implantat-Technik üblichen Metalle bestehen, sind die Klemmkörper vorzugsweise aus Kunststoff — unter Umständen durch Fasereinlagen verstärkt — gefertigt. Selbstverständlich können sie jedoch auch aus einem anderen der bewährten Implantat-Werkstoffe bestehen.

Im folgenden wird die Erfindung anhand von Ausführungsbeispielen im Zusammenhang mit der Zeichnung näher erläutert.

Fig. 1 gibt in einem Ausschnitt aus einer Seitenansicht den proximalen Bereich des neuen Schaftes wieder;

Fig. 2 ist der Schaft nach Fig. 1 im Schnitt II-II, schematisch eingesetzt in einen Femurknochen;

Fig. 3 zeigt in einer dem Schnitt III-III von Fig. 1 entsprechenden Darstellung ein zweites Ausführungsbeispiel;

Fig. 4 stellt einen Klemmkörper-Rohling dar, von dem

Fig. 5 und 6 je eine Ansicht von oben und von unten gesehen in der Richtung der Pfeile A und B sind.

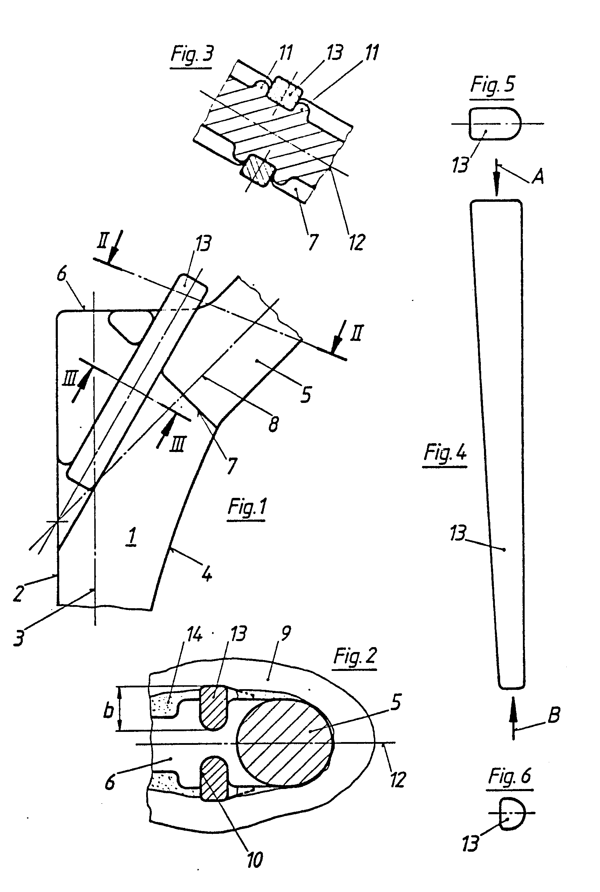

Die Ausführungsform nach Fig. 1 des nur in seinem proximalen Bereich gezeigten Schaftblattes 1 hat eine laterale Schmalseite 2, die in diesem Bereich parallel zur Längsmittelachse 3 verläuft. An ihrem oberen Ende schliesst eine im wesentlichen horizontal verlaufende Schulter 6, die den Übergang zu einem Prothesenhals 5 bildet, der den nicht gezeigten Gelenkkopf der Prothese trägt. Die mediale Schmalseite 4 ist ein Bogen, der ebenfalls in den Prothesenhals 5, dessen Achse mit 8 bezeichnet ist, übergeht.

Im Bereich der Blattseiten wird der Übergang zum Prothesenhals 5 gebildet durch einen vorspringenden Absatz 7, dessen Verlauf in etwa der Resektions- oder Schnittebene am Schenkelhals des Femurknochens 9 (Fig. 2) entspricht.

In beide Seiten des Baltes 1 des Schaftes sind bei der ersten Ausführungsform unter einem Winkel zur Längsmittelachse 3 verlaufende Nuten 10 vorhanden, die unmittelbar lateral des Prothesenhalses 5 beginnend sich zur lateralen Schmalseite 2 erstrecken. Soll das Schaftblatt 1 aus Festigkeitsgründen nicht geschwächt werden, so können die Nuten 10 durch schienenartige Vorsprünge 11 (Fig. 3), die aus den Oberflächen des Blattes 1 herausragen, ersetzt sein. Weiterhin können die Nuten 10 bzw. die Vorsprünge 11 bezüglich einer Mittel- oder Symmetrieebene 12 (Fig. 2 und 3) des Schaftes einander gegenüberliegen oder versetzt zueinander angeordnet sein.

Während der Implantations-Operation werden, nachdem der Schaft in die korrekte Lage gebracht und in dieser Lage gehalten ist, in die Nuten 10 bzw. zwischen die Vorsprünge 11 keilförmige Klemmkörper 13 eingeschlagen. Diese verkeilen sich dabei mit dem kortikalen Gewebe des Knochens 9 (Fig. 2), wobei unter Umständen vorhandenes spongiöses Gewebe 14 oder ein noch nicht ausgehärteter Knochenzement verdrängt werden. Die Keile 13 sichern auf diese Weise — besonders als Primärfixierung bei zementfreier Implantation —, dass der Schaft in der vorgesehenen Lage verbleibt und vor allem während des Gebrauchs relativ zum Knochen 9 keine Rotationsverschiebungen erfährt.

Ein Keil 13, der vorzugsweise aus Kunststoff, insbesondere aus Polyäthylen, besteht, ist in den Fig. 4-6 gezeigt; sein Rohling ist erheblich, beispielsweise 2 mal länger, als seine im Knochen 9 bzw. am Schaftblatt 1 verbleibenden Abschnitte. Diese doppelte Länge erlaubt dem Operateur, einen Abschnitt entsprechend der benötigten Keilbreite b (Fig. 2) aus der Länge des Rohlings auszuwählen; damit kann die Fertigung und Vorratshaltung verschieden breiter Keile 13 auf eine oder wenige Grössen unterschiedlicher Breite beschränkt werden.

**Patentansprüche**

1. Gerader, blattartiger Schaft für eine Gelenkendoprothese, insbesondere eine Hüftgelenkprothese, dessen Blattseiten sich vom distalen freien Ende zunächst symmetrisch zu einer Längsmittelachse konisch erweitern, ehe die laterale Schmalseite auf etwa 2/3 bis 3/4 der Schafthöhe — gemessen entlang der Längsmittelachse vom distalen Ende bis zu einer im wesentlichen horizontalen, zum Prothesenhals führenden Schulter — parallel zur Längsmittelachse oder auf sie zu verläuft, während die mediale Schmalseite in einem Bogen zum Prothesenhals geführt ist, dadurch gekennzeichnet, dass in den proximalen Bereich des Schaftes lateral des Prothesenhalses (5) in den Blattseiten (1) unter einem Winkel zur Längsmittelachse (3) verlaufende Führungen (10, 11) vorsehen sind, in die keilförmige Klemmkörper (13) eingeschoben sind, deren Länge mindestens das $1^1/_2$fache der Länge der Führungen (10, 11) beträgt.

2. Schaft nach Anspruch 1, dadurch gekennzeichnet, dass die Führungen als aus der Blattoberfläche herausragende Vorsprünge (11) ausgebildet sind.

**Claims**

1. A straight blade-like stem for a joint endoprosthesis, more particularly a hip joint replacement, the sides of the blade widening conically and initially symmetrically from the distal free end towards a longitudinal central axis before the lateral narrow side extends at about two-thirds to three-quarters of stem height — as measured along such axis from the distal end as far as a substantially horizontal shoulder extending to the neck of the implant — parallel or towards such axis, the medial narrow side being guided in an arc to such neck, characterised in that guides (10, 11) which extend at an angle to the longitudinal central axis (3) into the proximal zone of the stem laterally of the neck (5) of the implant are present in the blade sides (1) and receive wedgeshaped clamping members (13) whose length is at leat one and a half times the length of the guides (10, 11).

2. A stem according to claim 1, characterised in that the guides are projections (11) which project from the blade surface.

**Revendications**

1. Tige rectiligne du type spatule pour une endoprothèse articulée, en particulier une prothèse coxofémorale, dont les côtes de la spatule s'évasent tout d'abord tronconiquement à partir de l'extrémité libre distale, symétriquement par rapport à un axe médian longitudinal, avant que la face latérale étroite s'étende environ sur 2/3 à 3/4 de la hauteur de la tige — mesurée le long de l'axe médian longitudinal, de l'extrémité distale jusqu'à un épaulement sensiblement horizontal gagnant le col de la prothèse — parallèlement à l'axe médian longitudinal ou en direction de ce dernier, tandis que la face médiale étroite se raccorde par un arc au col de la prothèse, caractérisée par le fait que sont prévus des éléments de guidage (10, 11) qui s'étendent dans la région proximale de la tige latéralement par rapport au col (5) de la prothèse dans les côtés (1) de la spatule, selon un angle par rapport à l'axe médian longitudinal (3), éléments dans lesquels sont insérés des organes cunéiformes de serrage (13) dont la longueur représente au moins 1 fois et demi la longueur des éléments de guidage (10, 11).

2. Tige selon la revendication 1, caractérisée par le fait que les éléments de guidage sont réalisés sous la forme de saillies (11) dépassant de la surface de la spatule.

0 141 820

Fig. 3

Fig. 5

Fig. 4

Fig. 1

Fig. 2

Fig. 6

5